# EUROPEAN PATENT APPLICATION

(11) **EP 4 462 231 A1**
(43) Date of publication of application: **13.11.2024**
(21) Application number: 22920424.3
(22) Date of filing: 28.10.2022
(51) Int. Cl.: G06F 3/01, A61B 5/291, A61B 5/296, A61F 2/54, A61F 2/60, A61F 2/72, B25J 11/00

(54) **HUMAN AUGMENTATION PLATFORM DEVICE AND PHYSICAL ABILITY AUGMENTATION METHOD**

(30) Priority: 14.01.2022 JP 2022004463
(71) Applicant: NTT DOCOMO, INC., Tokyo 100-6150 (JP)
(72) Inventor: ISHIKAWA, Hironori, Tokyo 100-6150 (JP)
(74) Representative: Hoffmann Eitle
(86) International application number: PCT/JP2022/040489
(87) International publication number: WO 2023/135902

(57) **Abstract**

Provided is a human augmentation platform device 100, including: a physical information acquisition unit 110 that acquires physical information indicating a physical ability on a sensing side and a physical ability on a controlled side; a motion data acquisition unit 120 that acquires motion data on the sensing side; a control data generation unit 150 that generates control data on the controlled side on the basis of the physical information acquired by the physical information acquisition unit 110 and the motion data acquired by the motion data acquisition unit 120; and an actuation unit 160 that actuates a body on the controlled side on the basis of the control data. The control data generation unit 150 generates the control data within a range of the physical ability on the controlled side.

## Description

### Technical Field

The present disclosure relates to a human being augmentation platform device and a physical ability augmentation method for supporting augmentation of a physical ability of a human being.

### Background Art

The 3rd Generation Partnership Project (3GPP) has prepared a specification for the 5th generation mobile communication system (also called 5G, New Radio (NR), or Next Generation (NG)) and is also preparing next generation specifications called Beyond 5G, 5G Evolution, or 6G.

One of the technologies to focus on in the 6G era is human augmentation. Human augmentation can be broadly divided into "augmentation of physical ability", "augmentation of existence", "augmentation of perception", and "augmentation of cognitive abilities".

Among these, augmentation of physical ability refer to an approach to sense motion data, such as brainwaves and myoelectricity, from a brain and muscles of a human being, and actuating actual human muscles and an external skeleton. This can realize prosthetics for lost physical abilities, improvement of existing abilities, and acquisition of new abilities. Power assist suits, prosthetic arms and legs, and third arms (robotic arms) are considered (Non-Patent Literature 1).

### Citation List

### Non-Patent Literature

Non-patent literature 1: NTT DOCOMO, "DOCOMO 6G White Paper 4.0 Edition", [online], November 2021, Internet <URL: https://www.nttdocomo.co.jp/binary/pdf/corporate/technology/whitepap er_6g/DOCOMO_6G_White_PaperJP_20211108.pdf>

### Summary of the Invention

The physical ability augmentation described above has the following issues. Specifically, there may be a difference in physical abilities, e.g., possible range of motion, between a sensing side and an augmentation side (controlled side). There is also a difference in capacities (performance) of devices used for sensing and augmentation (control).

Thus, when motion data of a body on the sensing side is directly reflected to a human being or a robot on the augmentation side, there is an issue where an unreasonable burden is imposed on the augmentation side, or a physical ability on the augmentation side cannot be fully utilized.

Thus, the following disclosure is made in view of such situations and is intended to provide a human augmentation platform device and a physical ability augmentation method, capable of enhancing mutual cooperation between the sensing side and the augmentation side (controlled side).

An aspect of the present disclosure is a human augmentation platform device (human augmentation platform device 100), including a physical information acquisition unit (physical information acquisition unit 110) that acquires physical information indicating a physical ability on a sensing side and a physical ability on a controlled side, a motion data acquisition unit (motion data acquisition unit 120) that acquires motion data on the sensing side, a control data generation unit (control data generation unit 150) that generates control data on the controlled side on the basis of the physical information acquired by the physical information acquisition unit and the motion data acquired by the motion data acquisition unit, and an actuation unit (actuation unit 160) that actuates a body on the controlled side on the basis of the control data, wherein the control data generation unit generates the control data within a range of the physical ability on the controlled side.

An aspect of the present disclosure is a physical ability augmentation method, including a step of acquiring physical information indicating a physical ability on a sensing side and a physical ability on a controlled side, a step of acquiring motion data on the sensing side, a step of generating control data on the controlled side on the basis of the physical information and the motion data, and a step of actuating a body on the controlled side on the basis of the control data, wherein in the step of generating the control data, the control data is generated within a range of the physical ability on the controlled side.

### Brief Description of the Drawings

[FIG. 1] FIG. 1 is an overall schematic configuration diagram of a human augmentation system 10 according to a first embodiment.
[FIG. 2] FIG. 2 is a functional block configuration diagram of a human augmentation platform device 100 according to the first embodiment.
[FIG. 3] FIG. 3 is a diagram illustrating a configuration example of a physical information database ("DB") according to the first embodiment.
[FIG. 4] FIG. 4 is a diagram illustrating a configuration example of a motion DB according to the first embodiment.
[FIG. 5] FIG. 5 is a diagram schematically illustrating an operation example of the human augmentation platform device 100 according to the first embodiment.
[FIG. 6] FIG. 6 is a diagram illustrating a schematic operation flow of the human augmentation platform device 100 according to the first embodiment.
[FIG. 7] FIG. 7 is a diagram illustrating an augmentation motion example of a physical ability between a sensing side (body A) and a controlled side (body B) according to the first embodiment.
[FIG. 8] FIG. 8 is a diagram illustrating an example of a system configuration including the human augmentation platform device 100 according to a second embodiment.
[FIG. 9] FIG. 9 is a diagram illustrating designation pattern examples of sensing/actuation according to the second embodiment.
[FIG. 10] FIG. 10 is a diagram illustrating a sequence example of account registration and service subscription according to the second embodiment.
[FIG. 11] FIG. 11 is a diagram illustrating a sequence example (No. 1) related to body data registration according to the second embodiment.
[FIG. 12A] FIG. 12A is a diagram illustrating a sequence example (No. 2) related to body data registration according to the second embodiment.
[FIG. 12B] FIG. 12B is a diagram illustrating a sequence example (No. 3) related to body data registration according to the second embodiment.
[FIG. 13] FIG. 13 is a diagram illustrating an example of a real-time data transmission / reception sequence performed by a service management function and a data linkage control function according to the second embodiment.
[FIG. 14] FIG. 14 is a diagram illustrating a sequence example of skill data accumulation according to the second embodiment.
[FIG. 15] FIG. 15 is a diagram illustrating a sequence example of service registration according to the second embodiment.
[FIG. 16] FIG. 16 is a diagram illustrating a configuration example of data conversion elements in the data linkage control function according to the second embodiment.
[FIG. 17] FIG. 17 is a diagram illustrating an example of choice mapping according to the second embodiment.
[FIG. 18] FIG. 18 is a diagram illustrating an example of a hardware configuration of the human augmentation platform device 100.

### Description of Embodiments

Embodiments will be described below with reference to the drawings. Note that the same or similar reference numerals have been attached to the same functions and configurations, and the descriptions thereof is omitted as appropriate.

### First embodiment

### (1) Overall schematic configuration of a human augmentation platform device

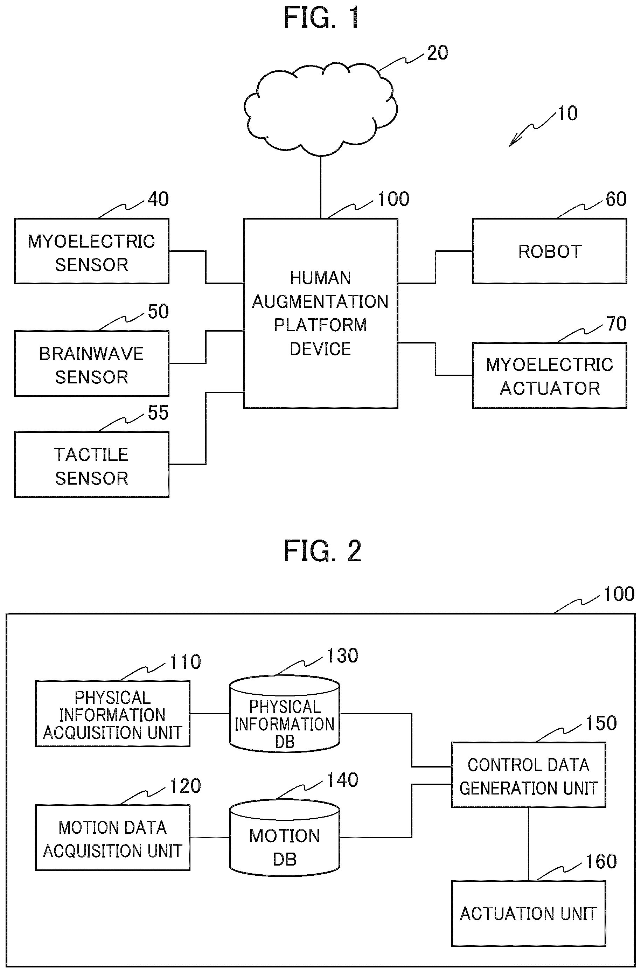

FIG. 1 is an overall schematic configuration diagram of a human augmentation system 10 according to the present embodiment. As illustrated in FIG. 1, the human augmentation system 10 includes a sensing side including a group of multiple sensors having different measurement objects, and an augmentation side (which may be referred to as a controlled side or an actuation side) where a robot or a human (human being) is a control object.

The human augmentation platform device 100 is connected to the sensing side and the augmentation side. The human augmentation platform device 100 may be connected to a communication network 20.

In the present embodiment, the human augmentation platform device 100 is connected to a myoelectric sensor 40 and a brainwave sensor 50. The human augmentation platform device 100 is connected to a robot 60 and a myoelectric actuator 70. In addition, the human augmentation platform device 100 may be connected to a tactile sensor 55.

The communication network 20 may include a wired network and a wireless network. The human augmentation platform device 100 may access other systems, databases, application services, and the like via the communication network 20.

The myoelectric sensor 40 is configured to be mounted on at least one of the limbs of a human being. Typically, the myoelectric sensor 40 can be mounted on an upper limb (arm) or a lower limb (leg) of a human being. However, the mounting object of the myoelectric sensor 40 need not necessarily be limited to a human being. The myoelectric sensor 40 is a sensor that detects an electrical signal generated during muscle contraction, and a common myoelectric (myoelectric potential) sensor can be used.

The brainwave sensor 50 is attached to the head of a human being. The brainwave sensor 50 is a sensor that detects potential fluctuations generated from the brain of a human being, and a common brainwave sensor can be used.

The tactile sensor 55 is a sensor capable of converting into data a tactile sense that a human being feels when touching an object. Typically, the tactile sensor 55 can convert into data a tactile sense of human being hands and feet in contact with an object of interest.

The robot 60 is a humanoid mechanical device that operates on the basis of a defined electrical signal. The robot 60, as with human beings, has at least one of an upper limb (arm) or a lower limb (leg). The robot 60 is not limited to have an arm (leg) and may have a portion such as a finger.

The myoelectric actuator 70 is configured to be attached to at least one of the limbs of a human being. Typically, the myoelectric actuator 70 can be attached to an upper limb (arm) or a lower limb (leg) of a human being. However, as with the myoelectric sensor 40, the object to which the myoelectric actuator 70 is attached need not necessarily be limited to a human being. The myoelectric actuator 70 can generate an electrical signal and can contract a muscle such as in a finger or an arm of a human being.

The human augmentation platform device 100 is connected to the sensing side and the augmentation side (controlled side) to realize human augmentation. Specifically, the human augmentation platform device 100 realizes augmentation of physical ability.

The augmentation of physical ability is an approach to actuate actual muscles and an external skeleton by sensing motion data, such as brainwaves and myoelectricity, mainly from a brain and muscles of a human being. The human augmentation platform device 100 acquires motion data of a body (body A) on the sensing side via the above-mentioned group of sensors.

The human augmentation platform device 100 converts the acquired motion data into control data using a conversion algorithm. The human augmentation platform device 100 operates (actuates) a body (body B) on the controlled side on the basis of control data generated by conversion of the motion data. By executing such processing by the human augmentation platform device 100, motion of the body A can be reflected on the body B. Note that the body B (or the body A) is not limited to a human being, and may include a robot.

The data (range) handled by the human augmentation platform device 100 is mainly myoelectricity (myoelectric potential) but may include brainwaves, biological sound waves, and motions (images). Myoelectricity will be described below as an example.

### (2) Functional block configuration of human augmentation platform device

FIG. 2 is a functional block configuration diagram of the human augmentation platform device 100. As illustrated in FIG. 2, the human augmentation platform device 100 includes a physical information acquisition unit 110, a motion data acquisition unit 120, a physical information DB 130, a motion DB 140, a control data generation unit 150, and an actuation unit 160.

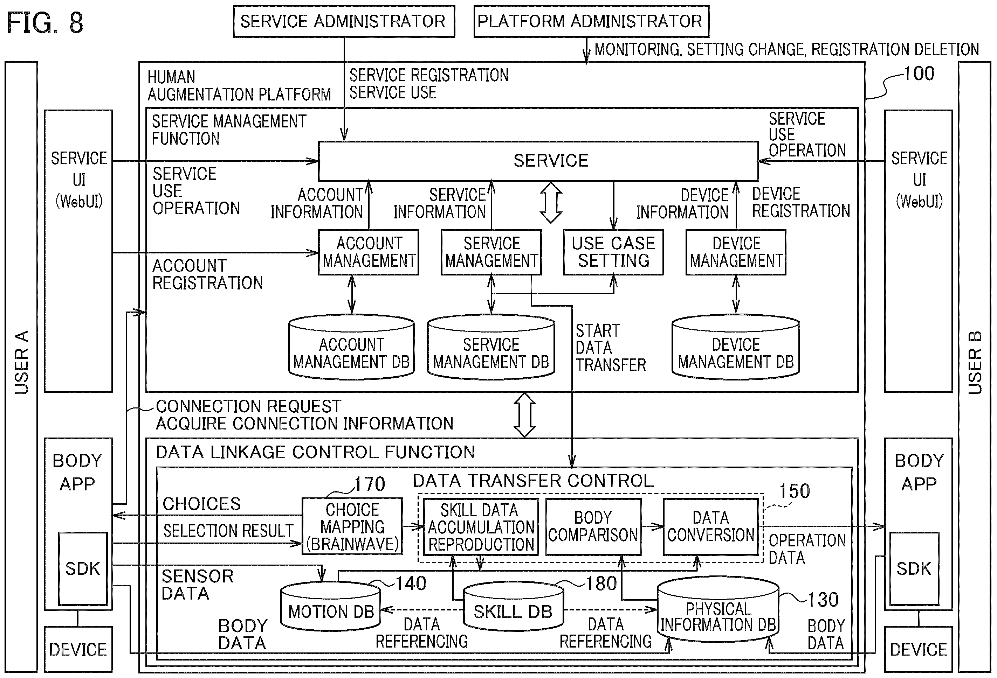

Note that only the main functional blocks relevant to the description of the embodiment are illustrated in FIG. 2, and that the human augmentation platform device 100 has other functional blocks (for example, a power supply unit or the like). FIG. 2 also illustrates a functional block configuration of the human augmentation platform device 100. Refer to FIG. 8 for the hardware configuration.

The physical information acquisition unit 110 acquires physical information indicating physical abilities on the sensing side and the controlled side. For example, the physical information acquisition unit 110 can acquire, as a physical ability, a possible range of motion of an arm of a human being (or robot) on the sensing side and on the controlled side. Note that a physical ability is not limited to an arm and may include a finger or a leg as objects. It is sufficient that a possible range of motion can be controlled by the myoelectric actuator 70, and it may be a range of flexion and extension, a range of twisting, or the like, with respect to a joint.

The physical information acquisition unit 110 may acquire physical information via the communication network 20, or may acquire physical information directly inputted to the human augmentation platform device 100. The acquired physical information is stored in the physical information DB 130.

The motion data acquisition unit 120 acquires motion data on the sensing side. Specifically, the motion data acquisition unit 120 acquires motion of a body on the sensing side through the myoelectric sensor 40 (the brainwave sensor 50 may be used, the same applies hereafter).

For example, the motion data acquisition unit 120 can acquire motion data (such as a range of flexion and extension with respect to a joint) of a human being arm on which the myoelectric sensor 40 is mounted. Specifically, the motion data acquisition unit 120 can generate motion data of a body portion on which the myoelectric sensor 40 is mounted, on the basis of an electric signal outputted from the myoelectric sensor 40. The acquired motion data is stored in the motion DB 140.

Note that in a case where the sensing side is a robot, the motion data acquisition unit 120 may generate motion data using motions (images) instead of the myoelectric sensor 40.

The physical information DB 130 stores physical information acquired by the physical information acquisition unit 110. Specifically, the physical information DB 130 can store physical information for each human being (or robot) subject to human augmentation.

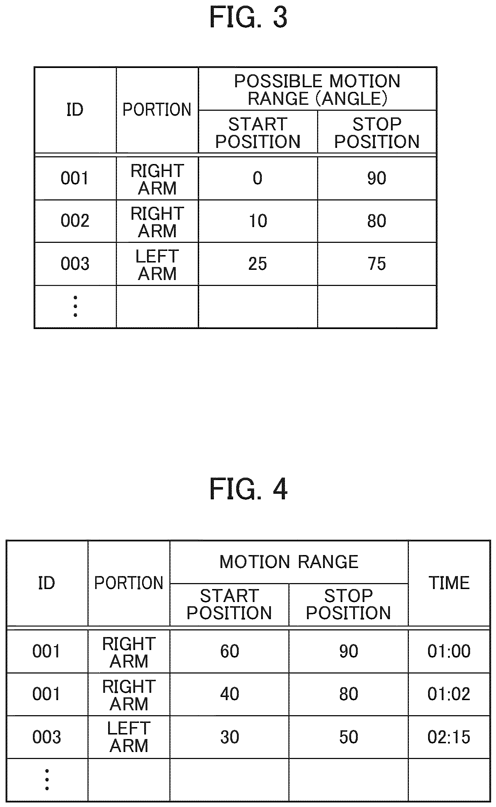

FIG. 3 illustrates a configuration example of a physical information DB. An ID is identification information for each human being (or robot). As illustrated in FIG. 3, the physical information DB may include a body portion and a possible range of motion. Note that FIG. 3 illustrates an example of the physical information DB and may include a physical ability other than the possible range of motion, such as weight that can be handled.

The motion DB 140 stores motion data acquired by the motion data acquisition unit 120. Specifically, the motion DB 140 can store motion data in a time series in which motion of a human being (or robot) is quantified.

FIG. 4 illustrates a configuration example of a motion DB. As with the physical information DB, an ID is identification information for each human being (or robot). As illustrated in FIG. 4, the motion DB may include a body portion and a motion range indicating the actual range of motion. Note that FIG. 4 illustrates an example of the motion DB and may include information other than the motion range, for example, a motion speed.

The control data generation unit 150 generates control data on the controlled side on the basis of physical information acquired by the physical information acquisition unit 110, and motion data acquired by the motion data acquisition unit 120. Specifically, the control data generation unit 150 generates control data on the basis of: physical information of a human being (or robot) on the sensing side and on the controlled side; and motion data generated from an electric signal acquired through the myoelectric sensor 40.

More specifically, the control data generation unit 150 acquires physical information of the body A on the sensing side, and physical information of the body B on the controlled side, from the physical information DB 130. The control data generation unit 150 acquires motion data of the body A from the motion DB 140. The control data generation unit 150 can determine a conversion algorithm for generating control data on the basis of the acquired physical information and motion data.

Here, the control data generation unit 150 can generate control data within a range of a physical ability on the controlled side (of the body B). A mechanism of conversion from motion data to control data includes: proportional interpolation of a possible motion range of a body, or of the minimum and maximum values of the ability of the myoelectric actuator 70 or the like; and performing control within a range of allowable values (stopping at the maximum value).

For example, the control data generation unit 150 may generate control data on the basis of a comparison result between the range of a physical ability on the sensing side (body A) and the range of a physical ability on the controlled side (body B) in such a manner that the physical ability on the controlled side is proportional to the physical ability on the sensing side. That is, even when there is a difference in physical abilities (for example, possible ranges of motion) between the body A and the body B, control data of the body B may be generated in proportion to the motion data of the body A while not exceeding the range of the physical ability of the body B. Note that a specific control example will be described below.

Alternatively, the control data generation unit 150 may generate control data on the basis of an allowable value of the physical ability on the controlled side (body B). For example, the maximum value of a possible motion range of an arm of the body B may be defined as an allowable value, and control data that is less than or equal to the allowable value may be generated.

In addition, when a portion of a limb on the sensing side (body A) differs from a portion of a limb on the controlled side (body B), the control data generation unit 150 can convert motion data of the portion on the sensing side to motion data of the portion on the controlled side. The control data generation unit 150 can generate control data on the basis of physical information of the body A and body B and the converted motion data. That is, in the human augmentation platform device 100, not only to operate (actuate) the same body portion, but also to actuate a different body portion (for example, the sensing side is an arm, and the controlled side is a leg), control data converted to motion of said body portion may be generated.

When the sensing side (body A) is a robot and the controlled side (body B) is a human being, the control data generation unit 150 may generate control data in such a manner that motion speed goes through an acceleration state, a constant state, and a deceleration state. Since motion of a robot (motor) is different from that of a human, the motion speed is changed according to motion of a human being.

When the upper or lower limb on the sensing side (body A) is one of the right and left (for example, right arm), and the upper or lower limb on the controlled side (body B) is the other of the right and left (for example, left arm), the control data generation unit 150 may convert a possible motion range on the sensing side into a possible motion range on the controlled side to generate control data.

For example, when two myoelectric sensors 40 are attached to the left arm of the body A and to a body A' different from the body A, and two myoelectric actuators 70 are attached to the left arm and right arm of the body B, control data of both arms of the body B may be generated on the basis of physical information of the body A and the body A', motion data of the body A and the body A', and physical information of the body B.

Here, when possible motion areas of the left arm and right arm of the body B overlap, that is, motion ranges based on motion data of body portions (left arm and right arm) overlap, the control data generation unit 150 may determine whether or not the left arm and the right arm come into contact. When the control data generation unit 150 determines that the left arm and the right arm come into contact, it may generate control data to avoid the contact. The control data to avoid the contact may be to simply stop motion before the contact, or may be to change a motion range or a motion timing to avoid the contact.

Further, for a possible motion area on the controlled side, stored information may be used when the physical information DB 130 stores information on both arms of the same body. Alternatively, on the basis of the assumption that a possible motion area of one arm (on the sensing side) is the same as a possible motion area of the other arm (on the controlled side), conversion into control data for the other arm may be performed. Note that the same processing may be applied not only to the arms but also to both legs and the like.

In order to cope with a difference in resolution due to capabilities of devices (robots, myoelectric actuators, etc.), or data loss due to communication errors, it is desirable that the control data generation unit 150 execute real-time processing instead of retransmission control.

When a new device is connected to the human augmentation platform device 100, the control data generation unit 150 may acquire base data on the device, determine a conversion algorithm for the device on the basis of the acquired physical information, and generate control data for the device.

The actuation unit 160 operates a body on the controlled side (body B) on the basis of control data generated by the control data generation unit 150. Specifically, the actuation unit 160 controls the robot 60 or the myoelectric actuator 70 (in the case of a human being) on the basis of the control data.

Note that in accordance with capabilities of the myoelectric actuator 70, conversion of displacement, force, velocity, and the like indicated by motion data acquired by the motion data acquisition unit 120 may be performed by applying a desired coefficient (multiple, etc.).

The actuation unit 160 may perform calibration for each combination of a sensor device (for example, a myoelectric sensor) and an actuation device (myoelectric actuator).

### (3) Operation of human augmentation platform device

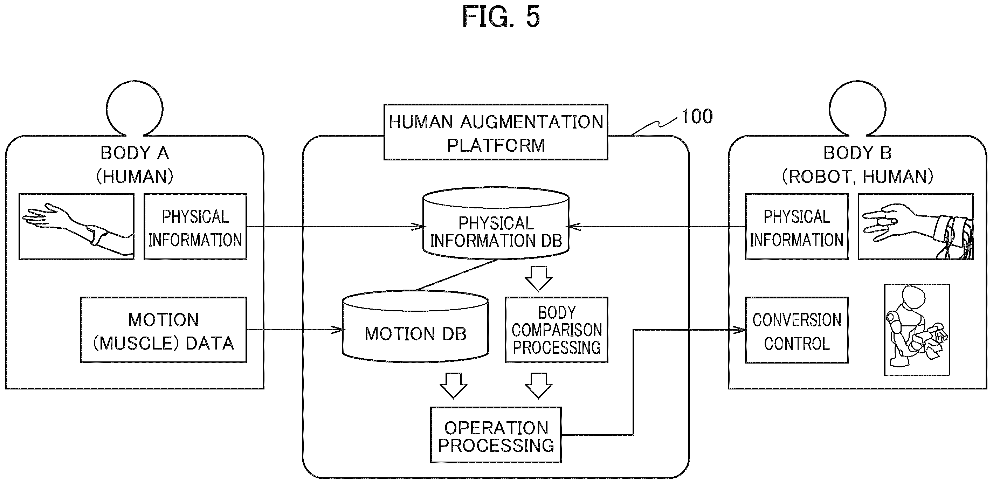

Next, an operation example of the human augmentation platform device 100 will be described. FIG. 5 schematically illustrates an operation example of the human augmentation platform device 100. In the operation example illustrated in FIG. 5, the sensing side (body A) is a right arm of a human being, and the controlled side (body B) is a right arm of a human being or a robot.

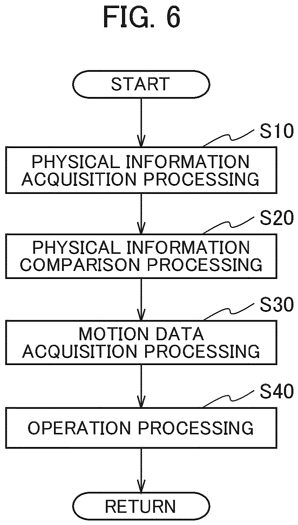

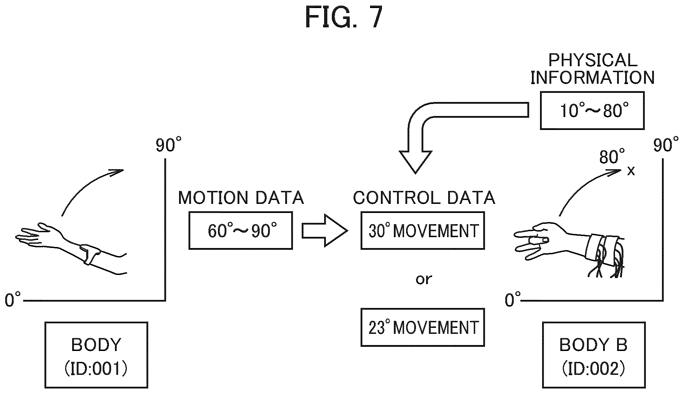

FIG. 6 illustrates a schematic operation flow of the human augmentation platform device 100. FIG. 7 illustrates an augmentation motion example of a physical ability between the sensing side (body A) and the controlled side (body B).

As illustrated in FIG. 6, the human augmentation platform device 100 acquires physical information on the sensing side and on the controlled side (S10). Specifically, the human augmentation platform device 100 acquires physical information on the body A and the body B. Here, the body A is assigned an ID of "001", and the body B is assigned an ID of "002" (see FIG. 7).

More specifically, the human augmentation platform device 100 acquires physical information (see FIG. 3) of the body A and the body B stored in the physical information DB 130.

Next, the human augmentation platform device 100 compares physical information of the body A (ID: 001) with physical information of the body B (ID: 002) (S20). The right arm of the body A (ID: 001) can move within a range from 0° to 90° (see FIG. 3). The right arm of the body B (ID: 002) can move within a range from 10° to 80° (see FIG. 3). The human augmentation platform device 100 compares physical information on the sensing side with physical information on the controlled side.

The human augmentation platform device 100 acquires motion data associated with motion of the body A (right arm) (S30). Specifically, the human augmentation platform device 100 acquires motion data indicating that the right arm of the body A, on which the myoelectric sensor 40 is mounted, has moved within a range from 60° to 90° (see FIG. 7). Note that 0° may be the horizontal direction, and 90° may be the vertical (vertical) direction. Further, processing in step S30 may be executed prior to processing in step S20.

The human augmentation platform device 100 executes processing for operating the body B on the controlled side (step S40). Specifically, the human augmentation platform device 100 generates control data for operating the body B on the basis of a comparison result between physical information on the sensing side and physical information on the controlled side, and motion data of the body A.

Specifically, the human augmentation platform device 100 generates control data of the body B on the basis of physical information of the body A (movable within a range from 0° to 90°), physical information of the body B (movable within a range from 10° to 80°), and motion data indicating that the right arm of the body A has moved within a range from 60° to 90° (that is, has moved 30°).

In this motion example, the control data of the body B may be control data of the myoelectric actuator 70 for moving the right arm of a human being by 30°, or control data for a robot for moving the right arm of the robot by 30°.

Alternatively, on the basis of the comparison result between physical information on the sensing side and physical information on the controlled side, control data may be generated in such a manner that the physical ability on the controlled side is proportional. For example, since the range of motion / the range of possible motion of the body A is (90 - 60) / (90 - 0) ≈ 0.33, control data in which a range of motion of the body B is (80 - 10) * 0.33 ≈ 23° may be generated (see FIG. 7).

### (4) Actions and effects

According to the above-described embodiment, the following actions and effects can be obtained. Specifically, the human augmentation platform device 100 can generate control data of the body B on the basis of: physical information on the sensing side (body A) and on the controlled side (body B); and motion data of the body A. Here, the human augmentation platform device 100 can generate control data within a range of a physical ability of the body B.

Thus, even when there is a difference in physical abilities, such as a possible motion range of an arm, between the sensing side (body A) and the controlled side (body B), it is possible to substantially suppress the occurrence of the issue where an unreasonable burden is imposed on the augmentation side, or a physical ability on the augmentation side cannot be fully utilized. That is, the human augmentation platform device 100 can enhance the mutual cooperation between the sensing side and the augmentation side (controlled side).

For example, when motion data of the body A is reflected on the body B as is, motion exceeding an allowable value of the body B may be reflected due to a difference in possible motion ranges or abilities, and thus there may be a significant burden on the body B. There can also be a difference in capabilities (performance) of devices used for sensing and augmentation (controlling). With the human augmentation platform device 100, even in the presence of such a difference in physical abilities or a difference in capabilities of devices, appropriate control data can be generated to achieve smooth and reasonable augmentation of physical ability.

In the present embodiment, the human augmentation platform device 100 can generate control data on the basis of a comparison result between a range of a physical ability on the sensing side (body A) and a range of a physical ability on the controlled side (body B) in such a manner that the physical ability on the controlled side is proportional to the physical ability on the sensing side. Thus, even when there is a difference in physical abilities between the sensing side and the controlled side, smooth and reasonable augmentation of physical ability can be realized.

Also, the human augmentation platform device 100 can generate control data on the basis of an allowable value of a physical ability on the controlled side. Thus, the augmentation of the physical ability can be realized without imposing an unreasonable burden on the controlled side.

In the present embodiment, when a portion of a limb on the sensing side differs from a portion of a limb on the controlled side, motion data of the portion on the sensing side can be converted into motion data of the portion on the controlled side. It is thus possible to realize more flexible augmentation of physical ability.

In the present embodiment, when the sensing side is a robot and the controlled side is a human being, the human augmentation platform device 100 can generate control data in which the operating speed goes through acceleration, constant, and deceleration states. It is thus possible to augment the physical ability in accordance with the actual movement of the human being.

### Second embodiment

In the present embodiment, basic functions of the human augmentation platform device 100 according to the first embodiment are further expanded, and an operation panel (user interface (UI)) is integrated with the human augmentation platform device 100 (human augmentation platform server). Here, the operation panel may be interpreted as a set of hardware and software for designating the sensing/actuation for data transmission by a user, and for performing start / stop operation of data transmission.

In the present embodiment, multiple data transmissions from sensing to actuation can be performed independently and simultaneously. Icons (devices) displayed on the operation panel can also be changed. In the human augmentation platform device 100 according to the present embodiment, service management and data linkage control functions are particularly enhanced.

### (1) System configuration

FIG. 8 illustrates a system configuration example including the human augmentation platform device 100 according to the present embodiment. As illustrated in FIG. 8, the human augmentation platform device 100 is roughly divided into a service management function and a data linkage control function. The human augmentation platform device 100 is connected to a service user interface (UI) and a body application corresponding to a user A, and those corresponding to a user B.

Hereinafter, functions provided by the human augmentation platform device 100 according to the present embodiment, and an outline of the components of the functions, will be described.

### (1.1) Data linkage control function

This part deals with human (human being) ubiquitous data. It has an interface with sensing / actuation, and can perform reception of body data, reception of sensor data from a sensing device, transmission to an actuation device, and the like. It also has functions for accumulation and reproduction of skill data, body comparison, and data conversion.

### (1.2) Body comparison

To fill in a difference between a body on the sensing side and a body on the actuation side (controlled side), two sets of body data are compared. As a comparison method, for example, a ratio calculation (B/A) of the actuation side / the sensing side, or setting of an upper limit value so as not to exceed a possible motion range of the body on the actuation side, can be used. A mechanism for setting a comparison method according to use cases is provided. Such a body comparison can provide a fail-safe mechanism considering the actuation side.

### (1.3) Data conversion

Sensor data transmitted from the sensing side is subjected to magnification conversion, target portion conversion (for example, from the right hand to the left hand), and the like. Data conversion processing is executed in the human augmentation platform device 100, and the result of conversion is transmitted to the actuation side. A mechanism for setting a conversion method according to use cases is provided. This can provide a mechanism by which a service provider can designate, in consideration of the actuation side, a magnification and a conversion method of information to be transmitted.

### (1.4) Skill data accumulation and reproduction

Data management for each user enables handling of accumulation and reproduction of sensor data. Past sensor data is managed in a motion database, and provision of a function of selecting and reproducing past data, together with a UI for selecting data from the database, is made possible. This can provide a mechanism for designating and reproducing any of accumulated skills.

### (1.5) Choice mapping (brainwaves)

In order to perform choice selection (for example, four choices or eight choices) based on brainwaves, a mechanism is provided for mapping choices and motions corresponding to the choices. Information on motions on the sensing side (icon image, text, and the like that illustrate the content of motion) is presented, converted into corresponding skill data on the basis of a choice (number) received from the sensing side, and transmitted to the actuation side. This can provide a mechanism for mapping motions, for example, by image selection.

### (1.6) Service management function

Apart from the data linkage control function, a service that can mutually combine, compare, and convert data, and a management function thereof are provided. This can set handling and conversion specifications of data for each service. Services can treat data as being mutually exclusive and independent.

### (1.7) Account management

In the human augmentation platform device 100, data can be accumulated for each user. Thus, an identifier (account) of a user is provided. An account belongs to one of the following categories: (1) a platform administrator, (2) a service administrator, and (3) a general user, and access rights are managed. An account management function for creating, editing, and deleting accounts is provided.

### (1.8) Device management

A device management function is provided to manage the types of devices permitted to connect to the human augmentation platform device 100. In this function, a mechanism is provided to control whether or not each type of device can connect to the human augmentation platform device 100. This enables only permitted devices to be connected to the human augmentation platform device 100.

### (1.9) Use case setting

A mechanism is provided to set a data transfer method (designation of sensing / actuation), a body comparison method, and a data conversion method for each service. This enables independent data linkage control for each service.

### (1.10) Service management

Users who can use a service, devices that can be connected, body applications (hereinafter, abbreviated as apps as appropriate), body comparison methods, and data conversion methods can be set for individual services. Each service has a UI which provides functions such as designating sensing / actuation, setting a body comparison method and a data conversion method, setting accumulation and reproduction of skill data, and indicating the start / end of data transmission. This enables the contents of sensing / actuation to be set for each service to be provided.

### (1.11) Body data, sensor data

The human augmentation platform device 100 handles the following human data. Such data may be interpreted as types of human data that can be transmitted to actuators.
• Joint angle and length of each portion of a body: data on a joint angle and the length of each portion

Data may be expanded to joint angles of the upper limb, joint angles of the trunk and the lower limb, and lengths of the upper limb and the lower limb.
• Grip strength: data on grip strength for hands and feet

A definition of grip strength for a foot may be added (used for walking, etc.)
• Tactile sense: the human tactile sense is converted into data to be manageable

Data definitions for the tactile sense of hands and feet may be provided.
• Emotions: human emotions are converted into data to be manageable. Emotions (tension levels) are converted into data to be manageable.

### (1.12) SDK

For device vendors, an SDK (development kit) capable of reducing the difficulty of connection with the human augmentation platform device 100 is provided. The SDK is preferably made unintelligible (encrypted) to increase the difficulty of analyzing program contents in order to prevent diversion for other purposes or falsification.

### (1.13) Device control app, reference code

To enhance the ease of device control app development by device vendors, a template of an app to be combined with an SDK may be provided simultaneously as a reference code. Device vendors can usually create a device control app on the basis of the reference code by adding a part for data transmission and reception with a device.

### (1.14) Test suite

A test suite is provided as a test environment (verification of test scenarios and test results) for the development of device control apps.

For scenario creation, sensor data is prepared in advance in text format (JSON), and thus it becomes possible to perform a test of transmitting the sensor data to the actuator. Testing of normal and quasi-normal systems, maximum, minimum, and abnormal values, and the like can be automated by sequentially testing various data sets. The evidence of the test results is output as a log, and it is possible to determine suitability of a developed device control app developed for the human augmentation platform device 100 by aggregating the log.

### (2) Details of data linkage control function and service management function

Next, details of the data linkage control function and the service management function (including components) described above will be explained.

### (2.1) Data linkage control function

The data linkage control function may include a function for receiving data from a sensing device and transmitting data to an actuation device, and a function for accumulating data received from the sensing device and reproducing the accumulated data on the actuation device.

The data linkage control function also includes a function for performing body comparison and data conversion on data to be transmitted to the actuation device. The data linkage control function may transmit and receive (transmit) data in accordance with an instruction from a service. An instruction from a service may include designating sensing / actuation required for data transmission, setting a body comparison method and a data conversion method, and setting accumulation and reproduction of skill data.

The following data transmission methods may be provided.
• Real-time transmission (data received from a sensing side is transmitted to an actuation)
• Accumulation of skill data (data is received from a sensing side and stored)
• Regeneration of skill data (accumulated data is read and transmitted to an actuation)

In the data linkage control function, multiple services can simultaneously and independently transmit data. A service provider can designate the magnification and conversion method of information to be transmitted considering the actuation side.

### (2.2) Body comparison

In order to fill in a body difference (size difference) between the sensing side and the actuation side, two sets of body data may be compared. The data comparison method may be configurable according to use cases.

The comparison method can be selected from, for example, "ratio calculation (B/A)" or "upper limit value setting (not exceeding B)". Here, A is body data on the sensing side, and B is body data on the actuation side. Note that other methods for comparing the body data may be applied. The service may be able to designate which comparison method is applied.

That is, in the present embodiment, the control data generation unit 150 of the human augmentation platform device 100 can generate control data on the basis of the ratio between physical information on the sensing side, and physical information on the actuation side (controlled side).

Conversion of operation data (motion data) on the basis of the body comparison data may be performed in the human augmentation platform device 100.

An application program interface (API) may be provided for registering body data required for body comparison. A method for inputting body data on the app side includes numerical input by a user, and input of data acquired from a sensing device.

The inputted body data is managed in the body DB for each user.

In the body DB, body IDs, users, body data, registration date and time, and the like can be managed. Table 1 illustrates a configuration example of the body DB.

**[Table 1]**

| Items | Descriptions |
|---|---|
| Body ID | ID to identify body data (non-duplicable, ID assignment at registration) |
| User ID | ID to identify user data |
| Body data | Height, age, possible range of motion at each joint, length of arms and legs |
| Registration date and time | Date and time of data registration |

### (2.3) Data conversion

Before the sensor data is transmitted to the actuation side, conversion processing may be performed according to use cases. The following conversion method may be provided according to use cases or formats of data to be handled.
• Scalar transformation: a magnification operation is performed on a single element of data.
· Matrix transformation: a portion conversion and a magnification operation are performed on multiple elements of data such as joint angles.
· Frequency characteristic transformation: an enhancement operation in a specific frequency band using fast Fourier transform (FFT), enhancement transform, and inverse fast Fourier transform (IFFT) is performed on waveform data.

The service may be able to designate which transformation method is applied.

### (2.4) Skill data accumulation and reproduction

Data transmitted from the sensing side may be accumulated, and skill data selected from the accumulated skill data may be transmitted to the actuation side. The data transmitted from the sensing side may be managed in the motion DB 140 (also called operation DB) for each user.

In the motion DB 140, history IDs, body IDs, date and time, and sensor data may be managed. Apart from the motion DB 140, a skill DB 180 is provided to manage the contents of accumulated data (user, date and time, content of motion, device, etc.) as skill data. Table 2 illustrates a configuration example of the motion DB.

**[Table 2]**

| Items | Descriptions |
|---|---|
| History ID | ID to identify sensor data in DB (non-duplicable, ID assignment at registration) |
| Body ID | ID to identify body data during data acquisition |
| Sensor data | Data acquired on sensing side such as angle at each joint, grip strength, tactile sense, and tension level |
| Date and time | Date and time of data registration |

In the skill DB 180, skill IDs, users, date and time, device IDs, motion contents, body IDs, history IDs, and the like may be managed. The substance of body data and sensor data is stored in the physical information DB 130 (also called body DB) and in the motion DB 140, respectively, and IDs for data reference are managed in the skill DB 180. Table 3 illustrates a configuration example of the skill DB.

**[Table 3]**

| Items | Descriptions |
|---|---|
| Skill ID | ID to identify skill data in DB (non-duplicable, ID assignment at registration) |
| Body ID | ID to identify body data during sensor data acquisition |
| History ID | ID to identify sensor data |
| Content of motion | String representing motion content of sensor data |
| Device ID | Device ID used during data acquisition |
| Date and time | Date and time of skill data registration |

A screen for registering, editing, and deleting skill data, and a screen for listing, selecting, and reproducing registered skill data may be provided as functions on the service side. In the skill data registration, a method for inputting and registering a skill content to accumulated sensor data, and a method for acquiring sensor data after inputting a skill content may be provided.

Thus, in the present embodiment, the human augmentation platform device 100 includes the skill DB 180 (skill data accumulation unit) for accumulating motion data acquired by the motion data acquisition unit (body app, sensor device, etc., may apply) as skill data arranged on the basis of predetermined features, and the control data generation unit 150 can generate control data for reproduction on the basis of skill data.

### (2.5) Choice mapping (brainwaves)

In order to perform choice selection (for example, four choices or eight choices) based on brainwaves, a mechanism is provided for mapping choices and motions corresponding to the choices. Motions corresponding to choices may be reproduction of skill data and may be transition to another choice.

Mapping information may include a choice (number) of multiple choices, a motion content corresponding to the choice, and an image (icon) illustrating the motion content. Mapping information of the choice may be returned in response to a request from the sensing side.

When the sensor data transmitted from the sensing side is a choice based on brainwaves, a motion corresponding to the choice may be performed. When the motion is reproduction of skill data, corresponding sensor data is transmitted to the actuation side. When the motion is transition to another mapping, new mapping information may be returned to the sensing side.

Thus, in the present embodiment, the human augmentation platform device 100 includes a choice mapping unit 170 that presents a choice of control data to a user, and the choice mapping unit 170 can specify skill data associated with a choice selected by the user. The control data generation unit 150 can generate control data for reproduction on the basis of the specified skill data.

### (2.6) Service management and use case setting

In service management, registration, editing, and deletion of services can be performed. Information about services is managed in a service database. Table 4 illustrates a configuration example of the service database.

**[Table 4]**

| Items | Descriptions |
|---|---|
| Service ID | Alphanumeric characters to identify service (non-duplicable, ID assignment at registration) |

| Service name | Name of service |
|---|---|
| Service subscriber | Information on user who can use service (including user connection status) |
| Connectable body app | Information on connectable body app (correspondence between sensing and actuation) |
| Body conversion method | Designate body comparison method such as ratio calculation and upper limit value setting |
| Data conversion method | Designate data conversion method such as portion replacement and magnification conversion |

Information about a service may include a service ID, a service name, information about a user (service subscriber) who can use the service, a connectable device body app, a body comparison method, and a data conversion method. The service may have a separate UI.

The UI of a service may provide functions such as designating sensing / actuation, setting a body comparison method, a data conversion method, and accumulation and reproduction of skill data, and starting / ending data transmission. Each service can manage users connected to the service. When the start of data transmission is selected on the UI, the service provides instruction to the data linkage control function according to the set use case (sensing / actuation, body comparison method, data conversion method, or the like).

### (2.7) Account management

Since users are identified when accessing the human augmentation platform device 100, it is possible to register, edit, and delete an account. As described above, account types provided may be (1) a platform administrator, (2) a service administrator, and (3) a general user.

Information about accounts is managed in an account database. Information about accounts may include user IDs, usernames, and account types. Table 5 illustrates a configuration example of the account DB.

**[Table 5]**

| Items | Descriptions |
|---|---|
| User ID | Alphanumeric characters to identify user (non-duplicable) |
| | Example: yamada.taro |
| Username | Name, nickname, or the like of user (string) |
| | Example: Taro Yamada |
| Account type | Type of account (platform administrator / service administrator / general user) |

Platform administrator and service administrator accounts can be registered, edited, and deleted by an existing platform administrator on an administrator registration screen. General user accounts can be registered, edited, and deleted by users themselves on a general user registration screen. They can also be edited and deleted on the administrator registration screen. To access the human augmentation platform device 100, it is necessary to log in by designating a registered account.

### (2.8) Device management

Registration, editing, and deletion of device information are possible in order to identify a device authorized to access the human augmentation platform device 100 (platform). Registration may be performed in combination of a device (hardware) and a body app (software). This is because there are multiple body apps with different functions on the same device.

Device information is managed in a device DB. Table 6 illustrates a configuration example of the device DB.

**[Table 6]**

| Items | Descriptions |
|---|---|
| Device ID | Alphanumeric characters to identify device (non-duplicable, ID assignment using in-platform program at device registration) |
| Device name | Name of device (HW) |
| Body app name | Name of body app (SW) |
| Type | Sensing / actuation |
| API key | Information (string) to identify on platform side an accessing device Issued on platform side during device registration |

Data about a device may include a device ID, a device name, a body app name, a type (sensing or actuation) and an API key. Device management may only be granted to a platform administrator. An API key may be issued when device information is registered.

The body app sets an API key when accessing the platform, and on the platform side, it is recognized by means of the API key that the device is authorized for access. The platform administrator notifies a body app developer of the issued API key, and the body app developer sets the notified API key to the body app.

### (2.9) Body data, sensor data

Body data may be interpreted as data relating to a user's body that needs to be compared with other bodies. Sensor data may be interpreted as data for conveying the state of a user's body. The following human-related data may be defined as body data and sensor data.
• Height, age
· Joint angles of upper and lower limbs, and lengths of upper and lower limb portions
· Grip strength of hands and feet
· Tactile sense of hands and feet
· Emotions (tension levels)

Such data may be interpreted as types of data about humans that can be transmitted to actuators.

### (2.10) SDK function

For device developers, an SDK (development kit) capable of reducing the difficulty of connection with the human augmentation platform device 100 may be provided. The SDK includes an API for accessing (connection, status notification, data transmission, data acquisition, etc.) the human augmentation platform device 100 from a body app. The SDK may include a callback mechanism for a body app to receive a notification from the human augmentation platform device 100.

### (3) Sensing / actuation designation patterns

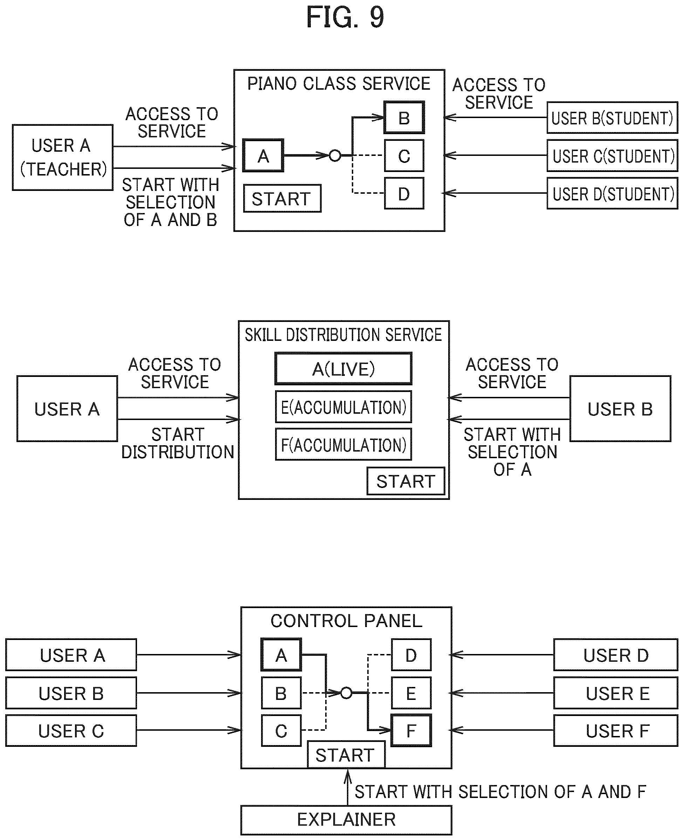

FIG. 9 illustrates pattern examples of sensing / actuation designation. As illustrated in FIG. 9, cases may be assumed where the initiator of actuation is the sensing side, the actuation side, or another operator.

For example, the sensing side may designate a distribution destination. The upper row of FIG. 9 illustrates a use case of a piano classroom where a teacher selects a student B who is in the classroom.

The actuation side may designate data to be used by itself. The middle row of FIG. 9 is a use case of live distribution of skills or distribution of accumulated skill data, and illustrates a pattern where the user B selects data A.

An operator other than the sensing side and the actuation side may provide designation. The lower row of FIG. 9 is a use case of a demonstration operation panel, and illustrates a pattern where an explainer starts data transfer through panel operation.

### (4) Sequence example

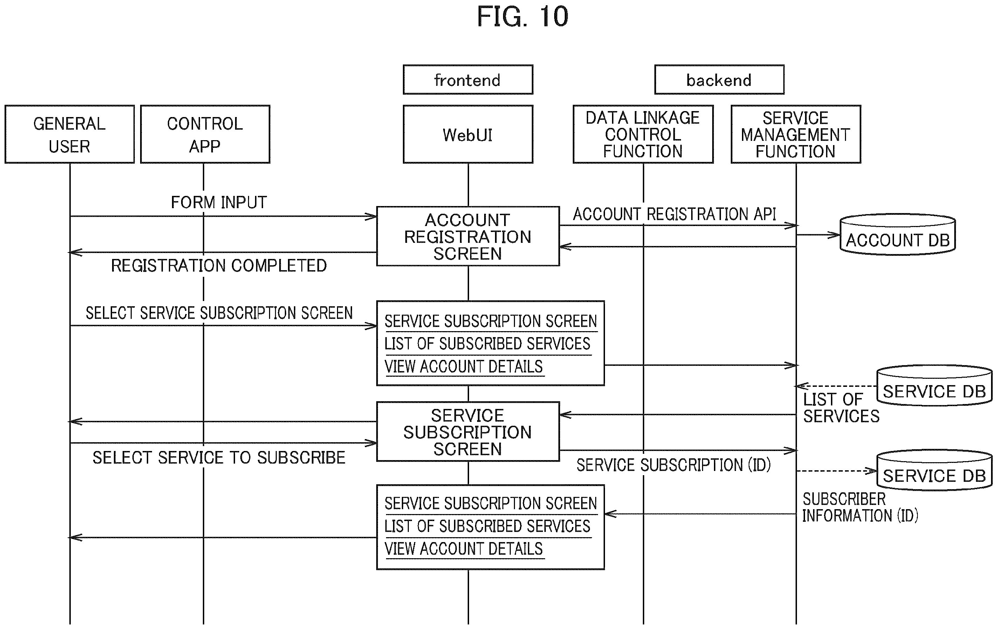

FIGS. 10 to 15 illustrate sequence examples of the data linkage control function and the service management function of the human augmentation platform device 100 according to the present embodiment.

FIG. 10 illustrates a sequence example of account registration and service subscription. As illustrated in FIG. 10, the service management function registers account information in the account database using an account registration API, and registers a service to be used by the account in the service database.

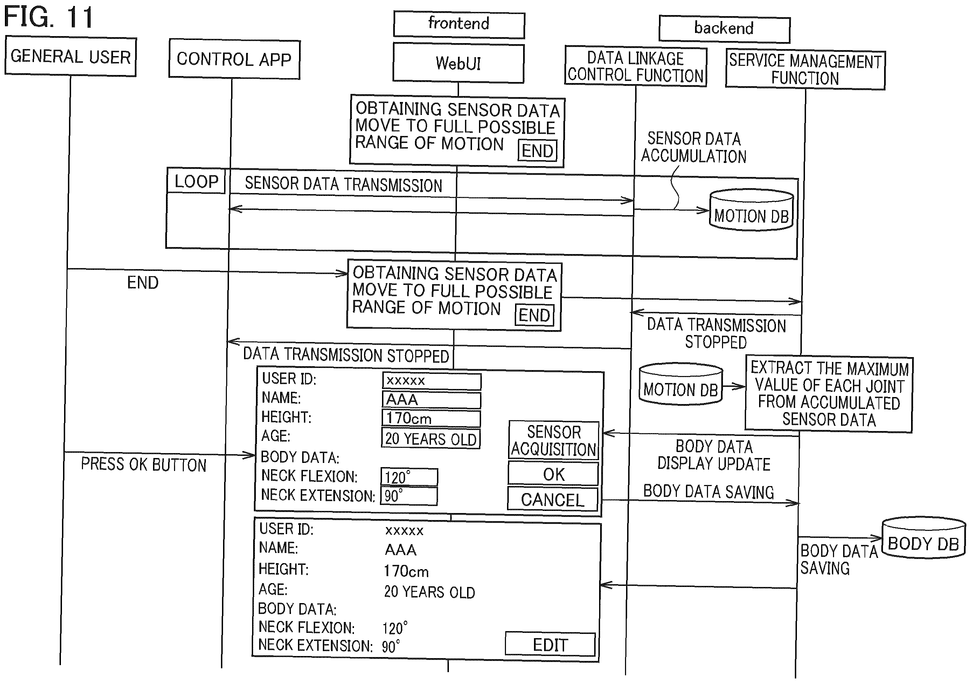

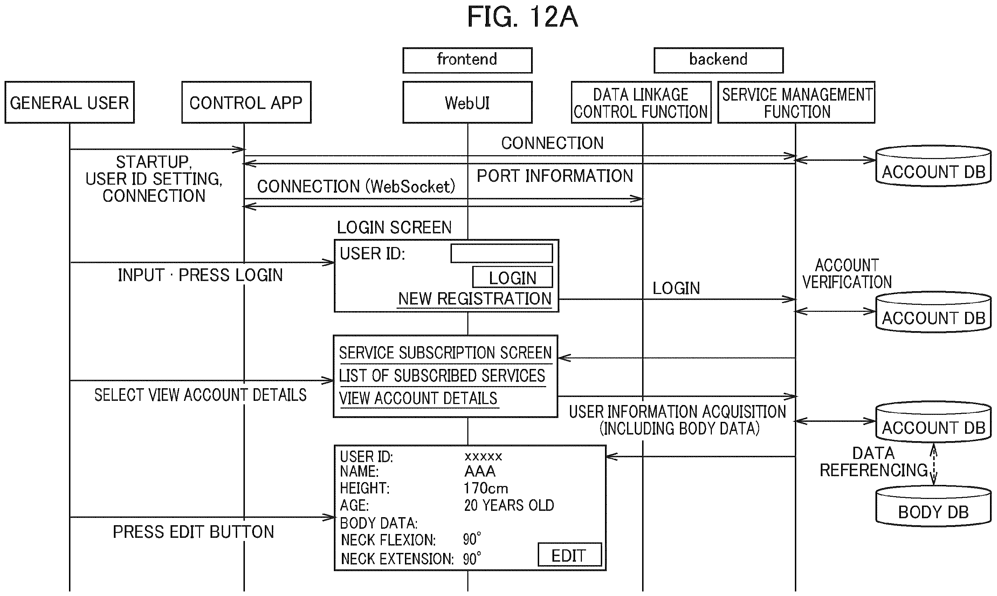

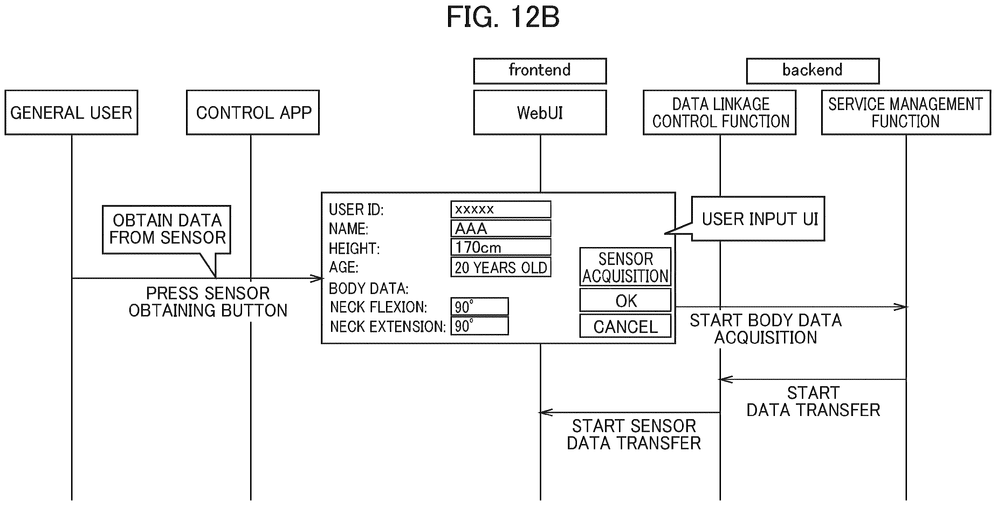

FIG. 11 illustrates a sequence example (part 1) related to body data registration. FIG. 12A illustrates a sequence example (part 2) related to the body data registration. FIG. 12B illustrates a sequence example (part 3) related to the body data registration.

As illustrated in FIG. 11, the service management function and the data linkage control function acquire body data (physical information) of a general user via a WebUI and a control application, and register the acquired body data in the body DB.

As illustrated in FIGS. 12A and 12B, the service management function and the data linkage control function can perform editing by the user of the body data registered in the body data DB. Thus, correlation of the body data of the user with a user ID (account DB), and the like is performed.

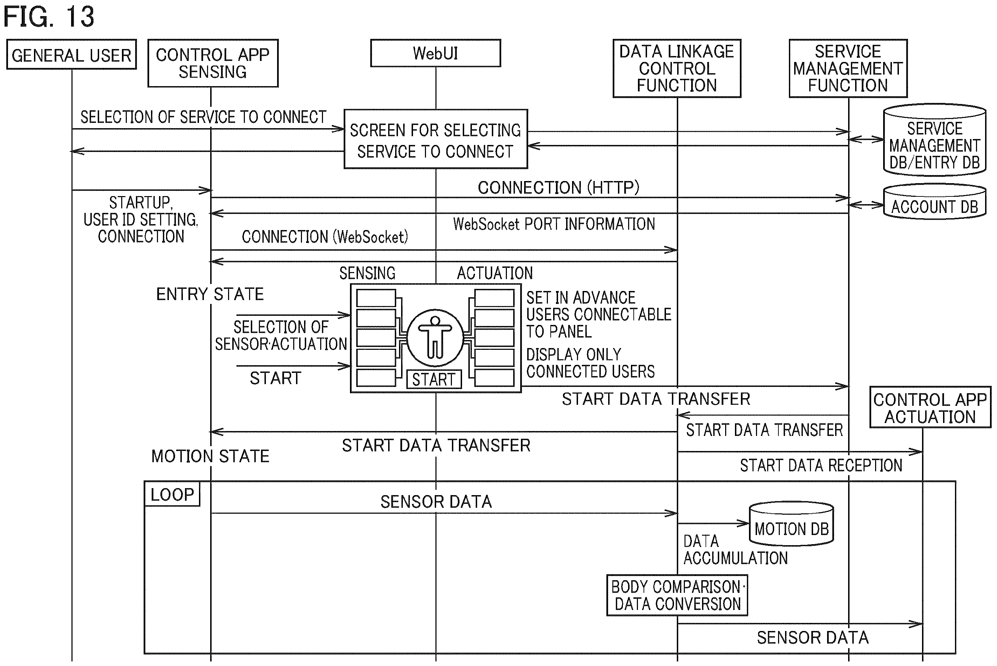

FIG. 13 illustrates a sequence example of a real-time data transmission / reception by the service management function and the data linkage control function. As illustrated in FIG. 13, the service management function and the data linkage control function can perform real-time sensor data transfer between the sensing side and the actuation side on the basis of a service selected by a general user.

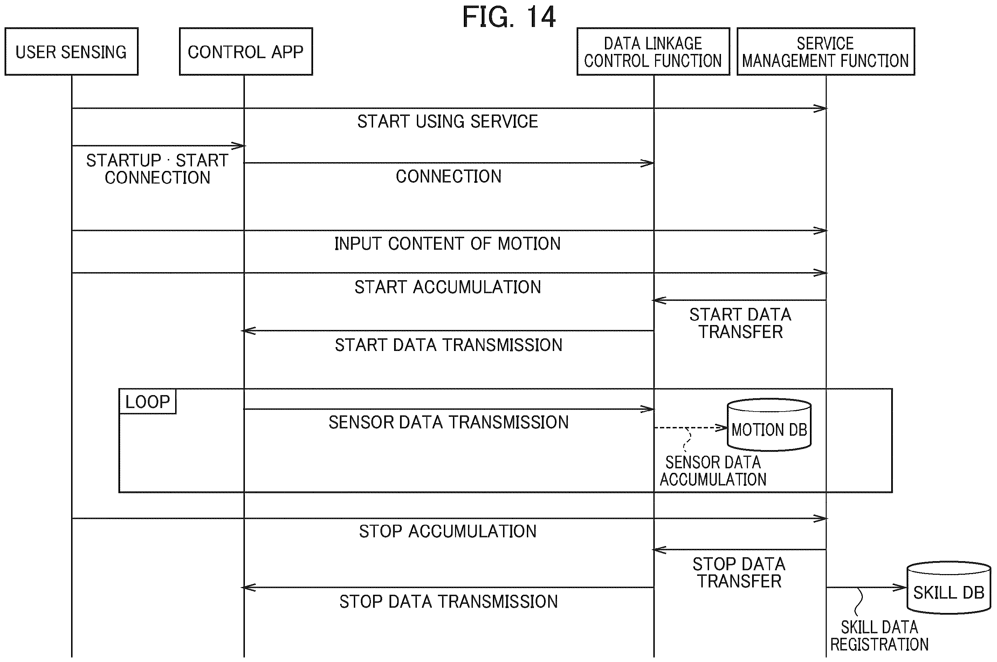

FIG. 14 illustrates a sequence example of skill data accumulation. As illustrated in FIG. 14, the service management function and the data linkage control function accumulate sensor data acquired through the control application in the motion DB. The service management function can register the sensor data as skill data in the skill DB.

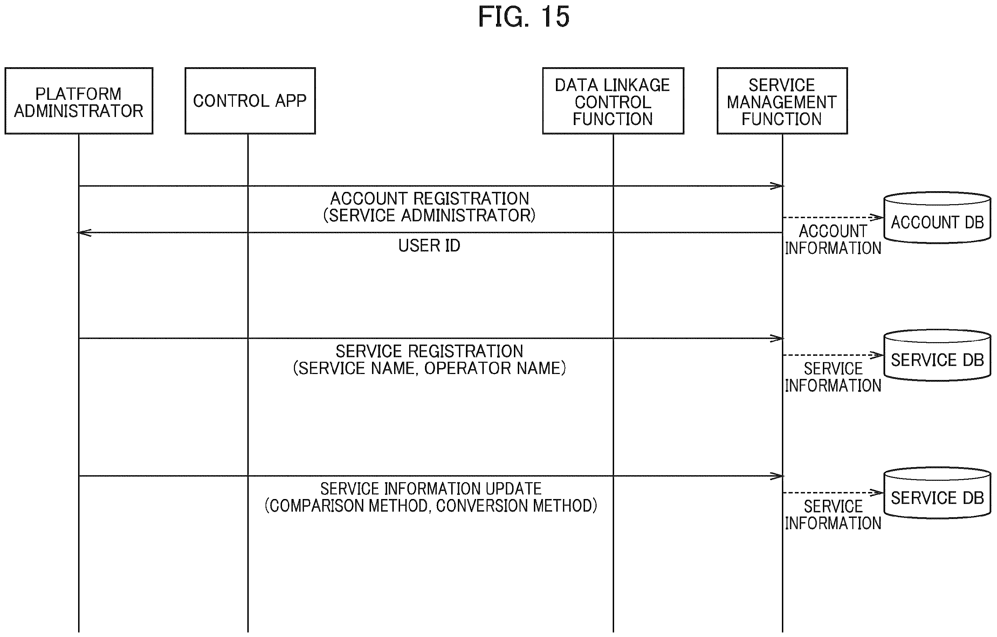

FIG. 15 illustrates a sequence example of service registration. As illustrated in FIG. 15, the service management function registers account information in the account DB and registers service information in the service DB. The service management function can register updated service information (such as a comparison method or a conversion method) in the service DB.

### (5) Data conversion details

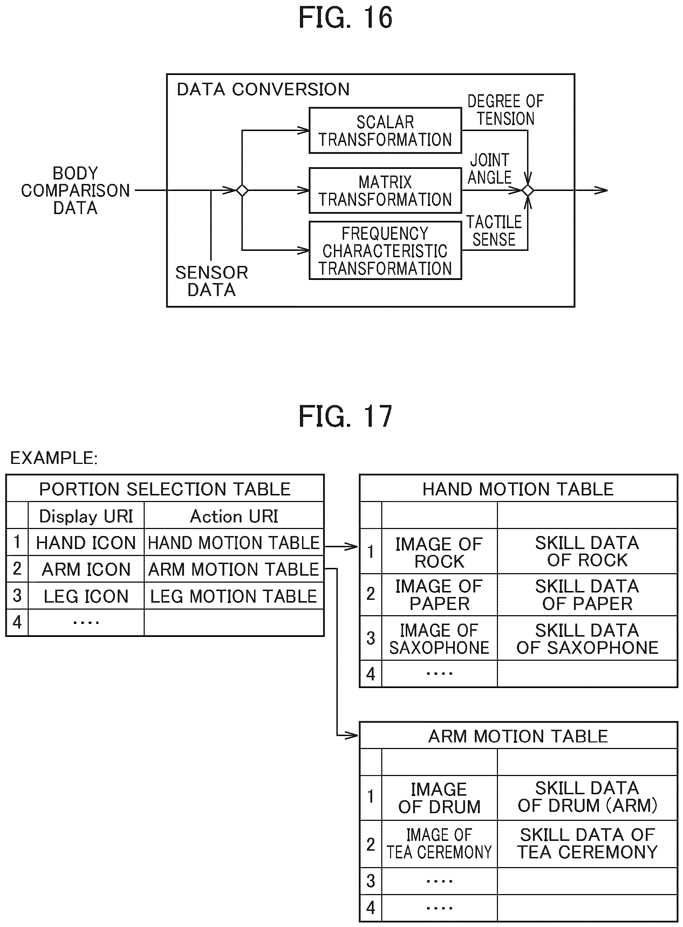

FIG. 16 illustrates a configuration example of data conversion elements in the data linkage control function. The human augmentation platform device 100 (control data generation unit 150) executes conversion processing of sensor data and transmits it to the actuation side. As illustrated in FIG. 16, one of the following may be applied to the conversion method of sensor data depending on use cases or formats of data to be handled.
· Scalar transformation: a magnification operation is performed on a single element of data.
· Matrix transformation: a portion conversion (such as right hand to left hand) and a magnification operation are performed on multiple elements of data such as joint angles.
· Frequency characteristic transformation: an enhancement operation in a specific frequency band using FFT, enhancement transform, and IFFT is performed on waveform data such as a tactile sense (sound data).

Note that when the number of data types handled increases, it may be possible to select from existing conversion methods.

### (6) Choice mapping (brainwaves)

FIG. 17 illustrates an example of choice mapping. In order to perform choice selection based on brainwaves, the human augmentation platform device 100 performs mapping between choices and motions corresponding to the choices.

As illustrated in FIG. 17, large classifications may be initially presented, and more details may be provided depending on a classification selected. It may also be possible to present hierarchical choices.

A mechanism may be provided to register the contents of choice mapping (table) via a service setting screen.

### (7) Actions and effects

In the human augmentation platform device 100 according to the present embodiment, the data linkage control function and the service management function can execute processing of body comparison and data conversion (Scalar transformation, matrix transformation, and frequency characteristic transformation), and further advanced mutual cooperation between the sensing side and the actuation side (controlled side) can be realized.

In addition, since the data linkage control function and the service management function can manage past sensor data (skill data) in the motion DB, past data can be selected and reproduced with the UI for selecting data from the motion DB, and more diverse and advanced human augmentation related services can be provided.

Furthermore, in the data linkage control function and the service management function, a mechanism for mapping choices and motions corresponding to the options is provided in order to perform choice selection (for example, four choices or eight choices) based on brainwaves. Thus, it becomes possible to convert a choice (number) received from the sensing side into relevant skill data and transmit it to the actuation side, thereby providing more diverse and advanced human augmentation related services.

### Other embodiments

Although the contents of the present invention have been described in accordance with the embodiments, the present invention is not limited to these descriptions, and it is obvious to those skilled in the art that various modifications and improvements thereof are possible.

For example, in the above-described embodiment, the myoelectric sensor 40 and the brainwave sensor 50 are connected to the human augmentation platform device 100, but sensor types are not limited to these, and a sensor or a device for detecting (acquiring) a specific action, such as sound waves (sounds), images (video), or a sport, may be connected.

The robot 60 and the myoelectric actuator 70 are connected to the human augmentation platform device 100, but objects of actuation are not limited to these, and a device or the like that is attached to a human being and simulates a specific motion, such as a sport, may be connected.

Block configuration diagrams (FIGS. 2 and 8) used in the description of the above-described embodiments illustrate blocks in units of functions. Those functional blocks (components) can be realized by any combination of at least one of hardware or software. A realization method for each functional block is not particularly limited. That is, each functional block may be realized by using one device combined physically or logically. Alternatively, two or more devices separated physically or logically may be directly or indirectly connected (for example, wired, or wireless) to each other, and each functional block may be realized by these plural devices. The functional blocks may be realized by combining software with the one device or the plural devices mentioned above.

Functions include judging, deciding, determining, calculating, computing, processing, deriving, investigating, searching, confirming, receiving, transmitting, outputting, accessing, resolving, selecting, choosing, establishing, comparing, assuming, expecting, considering, broadcasting, notifying, communicating, forwarding, configuring, reconfiguring, allocating (mapping), assigning, and the like. However, the functions are not limited thereto. For example, a functional block (component) that makes a transmitting function work may be called a transmitting unit or a transmitter. For any of the above, as described above, the realization method is not particularly limited.

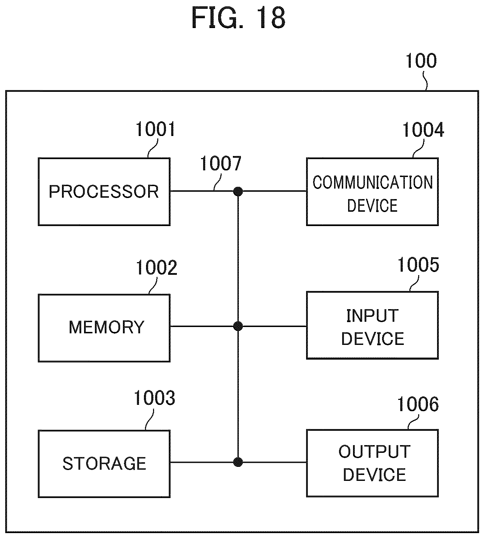

Further, the above-described the human augmentation platform device 100 may function as a computer that performs processing of a radio communication method of the present disclosure. FIG. 18 is a diagram illustrating an example of a hardware configuration of the human augmentation platform device 100. As illustrated in FIG. 18, the human augmentation platform device 100 may be configured as a computer device including a processor 1001, a memory 1002, a storage 1003, a communication device 1004, an input device 1005, an output device 1006, a bus 1007, and the like.

Furthermore, in the following description, the term "device" can be read as meaning circuit, device, unit, or the like. The hardware configuration of the device may include one or more devices illustrated in the figure or may not include some of the devices.

Each of the functional blocks of the device (FIGS. 2, 8) is implemented by means of any of hardware elements of the computer device or a combination of the hardware elements.

Each function in the device is realized by loading predetermined software (programs) on hardware such as the processor 1001 and the memory 1002 so that the processor 1001 performs arithmetic operations to control communication via the communication device 1004 and to control at least one of reading or writing of data on the memory 1002 and the storage 1003.

The processor 1001 operates, for example, an operating system to control the entire computer. The processor 1001 may be configured with a central processing unit (CPU) including interfaces with peripheral devices, control devices, arithmetic devices, registers, and the like.

Moreover, the processor 1001 reads a program (program code), a software module, data, and the like from at least one of the storage 1003 or the communication device 1004 into the memory 1002, and executes various processes according to these. As the program, a program causing the computer to execute at least part of the operation described in the above embodiment is used. Alternatively, various processes described above may be executed by one processor 1001 or may be executed simultaneously or sequentially by two or more processors 1001. The processor 1001 may be implemented by using one or more chips. Alternatively, the program may be transmitted from a network via a telecommunication line.

The memory 1002 is a computer readable recording medium and may be configured, for example, with at least one of a Read Only Memory (ROM), Erasable Programmable ROM (EPROM), Electrically Erasable Programmable ROM (EEPROM), Random Access Memory (RAM), or the like. The memory 1002 may be referred to as a register, cache, main memory (main storage device), and the like. The memory 1002 may store therein programs (program codes), software modules, and the like that can execute the method according to one embodiment of the present disclosure.

The storage 1003 is a computer readable recording medium. Examples of the storage 1003 include at least one of an optical disk such as Compact Disc ROM (CD-ROM), a hard disk drive, a flexible disk, a magneto-optical disk (for example, a compact disk, a digital versatile disk, Blu-ray (registered trademark) disk), a smart card, a flash memory (for example, a card, a stick, a key drive), a floppy (registered trademark) disk, a magnetic strip, or the like. The storage 1003 may be referred to as an auxiliary storage device. The recording medium may be, for example, a database including at least one of the memory 1002 or the storage 1003, a server, or other appropriate medium.

The communication device 1004 is hardware (transmission/reception device) capable of performing communication between computers via at least one of a wired network or a wireless network. The communication device 1004 is also referred to as, for example, a network device, a network controller, a network card, a communication module, and the like.

The communication device 1004 may include a high-frequency switch, a duplexer, a filter, a frequency synthesizer, and the like in order to realize, for example, at least one of Frequency Division Duplex (FDD) or Time Division Duplex (TDD).

The input device 1005 is an input device (for example, a keyboard, a mouse, a microphone, a switch, a button, a sensor, and the like) that accepts input from the outside. The output device 1006 is an output device (for example, a display, a speaker, an LED lamp, and the like) that outputs data to the outside. Note that, the input device 1005 and the output device 1006 may have an integrated configuration (for example, a touch screen).

Also, the respective devices such as the processor 1001 and the memory 1002 are connected to each other with the bus 1007 for communicating information. The bus 1007 may be constituted by a single bus or may be constituted by different buses for each device-to-device.

Further, the device may be configured to include hardware such as a microprocessor, a Digital Signal Processor (DSP), an Application Specific Integrated Circuit (ASIC), a Programmable Logic Device (PLD), and a Field Programmable Gate Array (FPGA). Some or all of these functional blocks may be realized by means of this hardware. For example, the processor 1001 may be implemented by using at least one of the above-described items of hardware.

Each aspect/embodiment described in the present disclosure may be applied to at least one of Long Term Evolution (LTE), LTE-Advanced (LTE-A), SUPER 3G, IMT-Advanced, the 4th generation mobile communication system (4G), the 5th generation mobile communication system (5G), Future Radio Access (FRA), New Radio (NR), W-CDMA (registered trademark), GSM (registered trademark), CDMA2000, Ultra Mobile Broadband (UMB), IEEE 802.11 (Wi-Fi (registered trademark)), IEEE 802.16 (WiMAX (registered trademark)), IEEE 802.20, Ultra-WideBand (UWB), Bluetooth (registered trademark), a system using any other appropriate system, or a next-generation system that is expanded based on these. Further, a plurality of systems may be combined (for example, a combination of at least one of LTE or LTE-A with 5G) and applied.

The order of the processing procedures, sequences, flowcharts, and the like of each aspect/embodiment described in the present disclosure may be exchanged as long as there is no contradiction. For example, the methods described in the present disclosure present the elements of the various steps by using an exemplary order and are not limited to the presented specific order.

Each of the aspects/embodiment described in the present disclosure may be used separately or in combination, or may be switched in accordance with the execution. In addition, notification of predetermined information (for example, notification of "is X") is not limited to being performed explicitly, and it may be performed implicitly (for example, without notifying the predetermined information).

Regardless of being referred to as software, firmware, middleware, microcode, hardware description language, or some other name, software should be interpreted broadly to mean instructions, an instruction set, code, a code segment, program code, a program, a subprogram, a software module, an application, a software application, a software package, a routine, a subroutine, an object, an executable file, an execution thread, a procedure, a function, and the like.

Further, software, instruction, information, and the like may be transmitted and received via a transmission medium. For example, when software is transmitted from a website, a server, or another remote source by using at least one of a wired technology (a coaxial cable, an optical fiber cable, a twisted pair cable, a Digital Subscriber Line (DSL), or the like) or a wireless technology (infrared light, microwave, or the like), then at least one of these wired or wireless technologies is included within the definition of the transmission medium.

Information, signals, or the like described in the present disclosure may be represented by using any of a variety of different technologies. For example, data, an instruction, a command, information, a signal, a bit, a symbol, a chip, or the like that may be mentioned throughout the above description may be represented by a voltage, a current, an electromagnetic wave, a magnetic field or magnetic particles, an optical field or photons, or any combination thereof.

It should be noted that the terms described in the present disclosure and terms necessary for understanding the present disclosure may be replaced with terms having the same or similar meanings. For example, at least one of a channel or a symbol may be a signal (signaling). A signal may also be a message. Further, a Component Carrier (CC) may be referred to as a carrier frequency, a cell, a frequency carrier, or the like.

The terms "system" and "network" used in the present disclosure can be used interchangeably.

Furthermore, information, parameters, and the like described in the present disclosure may be represented by an absolute value, may be represented by a relative value from a predetermined value, or may be represented by corresponding other information. For example, a radio resource may be indicated using an index.

In the present disclosure, the terms such as "base station (Base Station: BS)", "radio base station", "fixed station", "NodeB", "eNodeB (eNB)", "gNodeB (gNB)", "access point", "transmission point", "reception point", "transmission/reception point", "cell", "sector", "cell group", "carrier", "component carrier", and the like can be used interchangeably. A base station may also be referred to with a term such as a macro cell, a small cell, a femtocell, or a pico cell.

A base station can accommodate one or more (for example, three) cells (also referred to as sectors). In a configuration in which a base station accommodates a plurality of cells, the entire coverage area of the base station can be divided into a plurality of smaller areas. In each of the smaller areas, a communication service can be provided by a base station subsystem (for example, a small base station for indoor use (Remote Radio Head: RRH)).

The term "cell" or "sector" refers to a part or all of the coverage area of at least one of a base station or a base station subsystem that performs a communication service in this coverage.

In the present disclosure, the terms such as "mobile station (Mobile Station: MS)", "user terminal", "user equipment (User Equipment: UE)", and "terminal" can be used interchangeably.

A mobile station may be referred to as a subscriber station, a mobile unit, a subscriber unit, a wireless unit, a remote unit, a mobile device, a wireless device, a wireless communication device, a remote device, a mobile subscriber station, an access terminal, a mobile terminal, a wireless terminal, a remote terminal, a handset, a user agent, a mobile client, a client, or some other suitable terms by those skilled in the art.

At least one of a base station or a mobile station may be called a transmitting device, a receiving device, a communication device, or the like. Note that at least one of a base station or a mobile station may be a device mounted on a moving body, a moving body itself, or the like. The moving body may be a vehicle (for example, a car, an airplane, or the like), an unmanned moving body (a drone, a self-driving car, or the like), or a robot (manned type or unmanned type). At least one of a base station or a mobile station also includes a device that does not necessarily move during the communication operation. For example, at least one of a base station or a mobile station may be an Internet of Things (IoT) device such as a sensor.

Also, a base station in the present disclosure may be read as meaning a mobile station (user terminal, hereinafter, the same). For example, each aspect/embodiment of the present disclosure may be applied to a configuration in which communication between a base station and a mobile station is replaced with communication between a plurality of mobile stations (which may be called Device-to-Device (D2D), Vehicle-to-Everything (V2X), or the like). In this case, the mobile station may have the function of a base station. In addition, words such as "uplink" and "downlink" may also be read as meaning words corresponding to inter-terminal communication (for example, "side"). For example, an uplink channel, a downlink channel, or the like may be read as meaning a side channel (or a side link).

Similarly, the mobile station in the present disclosure may be read as meaning a base station. In this case, the base station may have the function of the mobile station.

The terms "connected", "coupled", or any variations thereof mean any direct or indirect connection or coupling between two or more elements, and can include that one or more intermediate elements are present between two elements that are "connected" or "coupled" to each other. The coupling or connection between the elements may be physical, logical, or a combination thereof. For example, "connection" may be read as meaning "access". In the present disclosure, two elements can be "connected" or "coupled" to each other by using at least one of one or more wires, one or more cables, or one or more printed electrical connections, and as some non-limiting and non-exhaustive examples, by using electromagnetic energy having wavelengths in the radio frequency domain, a microwave region, and a light (both visible and invisible) region, and the like.

A Reference Signal may be abbreviated as RS and may be called a pilot (Pilot) according to applicable standards.

As used in the present disclosure, the phrase "based on" does not mean "based only on" unless explicitly stated otherwise. In other words, the phrase "based on" means both "based only on" and "based at least on".

"Means" in the configuration of each device above may be replaced with "unit", "circuit", "device", and the like.

Any reference to elements using a designation such as "first", "second", or the like used in the present disclosure generally does not limit the amount or order of those elements. Such designations can be used in the present disclosure as a convenient method to distinguish between two or more elements. Thus, the reference to the first and second elements does not imply that only two elements can be adopted, or that the first element has to precede the second element in some or the other manner.

In the present disclosure, the used terms "include", "including", and variants thereof are intended to be inclusive in a manner similar to the term "comprising". Furthermore, the term "or" used in the present disclosure is intended not to be an exclusive-OR.

Throughout the present disclosure, for example, during translation, if articles such as a, an, and the in English are added, the present disclosure may include that a noun following these articles is used in plural.

As used in this disclosure, the term "determining" may encompass a wide variety of actions. "Determining" includes deeming that determining has been performed by, for example, judging, calculating, computing, processing, deriving, investigating, searching (looking up, search, inquiry) (for example, searching in a table, database, or another data structure), ascertaining, and the like. In addition, "determining" can include deeming that determining has been performed by receiving (for example, receiving information), transmitting (for example, transmitting information), inputting (input), outputting (output), access (accessing) (for example, accessing data in a memory), and the like. In addition, "determining" can include deeming that determining has been performed by resolving, selecting, choosing, establishing, comparing, and the like. That is, "determining" may include deeming that "determining" regarding some action has been performed. Moreover, "determining" may be read as meaning "assuming", "expecting", "considering", and the like.

In the present disclosure, the wording "A and B are different" may mean "A and B are different from each other". It should be noted that the wording may mean "A and B are each different from C". Terms such as "separate", "couple", or the like may also be interpreted in the same manner as "different".

### Note

The above-described disclosure may be expressed as follows.

According to a first aspect, a human augmentation platform device includes: a physical information acquisition unit that acquires physical information indicating a physical ability on a sensing side and a physical ability on a controlled side; a motion data acquisition unit that acquires motion data on the sensing side; a control data generation unit that generates control data on the controlled side on the basis of the physical information acquired by the physical information acquisition unit and the motion data acquired by the motion data acquisition unit; and an actuation unit that actuates a body on the controlled side on the basis of the control data, wherein the control data generation unit generates the control data within a range of the physical ability on the controlled side.

According to a second aspect, in the first aspect, the control data generation unit generates the control data on the basis of a comparison result between a range of the physical ability on the sensing side and the range of the physical ability on the controlled side in such a manner that the physical ability on the controlled side is proportional to the physical ability on the sensing side.

According to a third aspect, in the first or second aspect, the control data generation unit generates the control data on the basis of an allowable value of the physical ability on the controlled side.

According to a fourth aspect, in the first to third aspects, the control data generation unit generates the control data on the basis of a ratio between the physical information on the sensing side and the physical information on the controlled side.

According to a fifth aspect, in the first to fourth aspects, when a portion of a limb on the sensing side differs from a portion of a limb on the controlled side, the control data generation unit converts motion data on the portion on the sensing side into motion data on the portion on the controlled side, and generates the control data on the basis of the physical information and the motion data that has been converted.

According to a sixth aspect, in the first to fifth aspects, a skill data accumulation unit that accumulates the motion data acquired by the motion data acquisition unit, as skill data arranged on the basis of predetermined features is included, wherein the control data generation unit generates the control data for reproduction on the basis of the skill data.

According to a seventh aspect, in the first to sixth aspects, a choice mapping unit that presents a choice of the control data to a user is included, wherein the choice mapping unit specifies the skill data associated with the choice selected by the user, and the control data generation unit generates the control data for reproduction on the basis of the skill data that has been specified.

According to an eighth aspect, in the first to seventh aspects, the control data generation unit, when a limb on the controlled side includes multiple portions and when motion ranges based on the motion data of each of the portions overlap, determines whether or not one of the portions will come into contact with another portion, and when it is determined that contact will be made, generates the control data to avoid the contact.

Although the present disclosure has been described in detail above, it will be obvious to those skilled in the art that the present disclosure is not limited to the embodiments described in the present disclosure. The present disclosure can be implemented as modifications and variations without departing from the spirit and scope of the present disclosure as defined by the claims. Therefore, the description of the present disclosure is for the purpose of illustration, and does not have any restrictive meaning to the present disclosure.

### Reference Signs List

10 Human augmentation system
20 Communication network
40 Myoelectric sensor
50 Brainwave sensor
55 Tactile sensor
60 Robot
70 Myoelectric actuator
100 Human augmentation platform device
110 Physical information acquisition unit
120 Motion data acquisition unit
130 Physical information DB
140 Motion DB
150 Control data generation unit
160 Actuation unit
170 Choice mapping unit
180 Skill DB
1001 Processor
1002 Memory
1003 Storage
1004 Communication device
1005 Input device
1006 Output device
1007 Bus

## Claims

1. A human augmentation platform device, comprising:
a physical information acquisition unit that acquires physical information indicating a physical ability on a sensing side and a physical ability on a controlled side;
a motion data acquisition unit that acquires motion data on the sensing side;
a control data generation unit that generates control data on the controlled side on the basis of the physical information acquired by the physical information acquisition unit and the motion data acquired by the motion data acquisition unit; and
an actuation unit that actuates a body on the controlled side on the basis of the control data, wherein
the control data generation unit generates the control data within a range of the physical ability on the controlled side.

2. The human augmentation platform device according to claim 1, wherein the control data generation unit generates the control data on the basis of a comparison result between a range of the physical ability on the sensing side and the range of the physical ability on the controlled side in such a manner that the physical ability on the controlled side is proportional to the physical ability on the sensing side.

3. The human augmentation platform device according to claim 1, wherein the control data generation unit generates the control data on the basis of an allowable value of the physical ability on the controlled side.

4. The human augmentation platform device according to claim 1, wherein the control data generation unit generates the control data on the basis of a ratio between the physical information on the sensing side and the physical information on the controlled side.

5. The human augmentation platform device according to claim 1, wherein
when a portion of a limb on the sensing side differs from a portion of a limb on the controlled side, the control data generation unit
converts motion data on the portion on the sensing side into motion data on the portion on the controlled side, and
generates the control data on the basis of the physical information and the motion data that has been converted.

6. The human augmentation platform device according to claim 1, further comprising:
a skill data accumulation unit that accumulates the motion data acquired by the motion data acquisition unit, as skill data arranged on the basis of predetermined features, wherein
the control data generation unit generates the control data for reproduction on the basis of the skill data.

7. The human augmentation platform device according to claim 6, further comprising:
a choice mapping unit that presents a choice of the control data to a user, wherein
the choice mapping unit specifies the skill data associated with the choice selected by the user, and
the control data generation unit generates the control data for reproduction on the basis of the skill data that has been specified.

8. The human augmentation platform device according to claim 1, wherein
the control data generation unit,
when a limb on the controlled side includes multiple portions, and when motion ranges based on the motion data of each of the portions overlap, determines whether or not one of the portions will come into contact with another portion, and
when it is determined that contact will be made, generates the control data to avoid the contact.

9. The human augmentation platform device according to claim 1, wherein when the sensing side is a robot and the controlled side is a human being, the control data generation unit generates the control data in such a manner that a motion speed goes through an accelerated state, a constant state, and a decelerated state.

10. A physical ability augmentation method, comprising:
a step of acquiring physical information indicating a physical ability on a sensing side and a physical ability on a controlled side;
a step of acquiring motion data on the sensing side;
a step of generating control data on the controlled side on the basis of the physical information and the motion data; and
a step of actuating a body on the controlled side on the basis of the control data, wherein
in the step of generating the control data, the control data is generated within a range of the physical ability on the controlled side.
